# EUROPEAN PATENT SPECIFICATION

(11) **EP 2 042 608 B1**
(45) Date of publication and mention of the grant of the patent: **03.02.2010**
(21) Application number: 07016622.8
(22) Date of filing: 24.08.2007
(51) Int. Cl.: C12P 19/56

(54) **Genetically modified Streptomyces strains for biotransformations in anthracycline production**
Genetisch modifizierte Streptomyces-Stämme für Biotransformationen bei der Anthracyclin-Herstellung
Souches de Streptomyces modifiées génétiquement pour biotransformations dans la production d'anthracyclines

(43) Date of publication of application: 01.04.2009
(73) Proprietor: W.C. Heraeus GmbH, 63450 Hanau (DE)
(72) Inventor: Lambert, Michael, Dr.,, 63486 Gruendau (DE); Ylihonko, Kristiina, Dr., 20760 Piispanristi (FI); Holmbaeck, Maria, 21500 Piikkioe (FI)
(74) Representative: Kühn, Hans-Christian

(56) References cited:
- N.N.: "Powerful stuff" SPEC CHEM ONLINE, 15 June 2006 (2006-06-15), pages 1-7, XP002467571 Retrieved from the Internet: URL:http://www.specchemonline.com/shownews .asp?secid=7&nav=1&newstype=&key=&page=&ne wsid=11268> [retrieved on 2008-02-04]
- SALAS ET AL: "Genetic manipulation of antitumor-agent biosynthesis to produce novel drugs" TIBTECH (TRENDS IN BIOTECHNOLOGY), vol. 16, 1998, pages 475-482, XP002467572
- GIARDINA ET AL: "A putative membrane protein and an ABC-transporter of Planobispora Rosea induce antimicrobial activity in Streptomyces Lividans" III CONGRESSO ANNUALE DIPARTIMENTO DI BIOLOGIA CELLULARE E DELLO SVILUPPO, UNIVERSITÀ DI PALERMO, DICEMBRE 2005, 2005, page 1, XP002467629 Retrieved from the Internet: URL:www.unipa.it/dipbio/congresso2005/Giar dina_Puglia_dbcs_2005.pdf> [retrieved on 2008-02-05]
- HAUTALA ET AL: "Studies on a second and third ring cyclization in anthracycline biosynthesis" THE JOURNAL OF ANTIBIOTICS, vol. 56, 2003, pages 143-153, XP009088730

## Description

### Field of the invention

The present invention relates to improved microbial strains of the genera Streptomyces and to their use in a biotransformation process for improving the yields of anthracycline antitumor antibiotics, particularly epirubicin and idarubicin.

### Background of the invention

Daunomycins are a group of antitumor antibiotics produced by several *Streptomyces sp.,* such as *S. peucetius, S. coerulorubidus, S. griseus, Streptomyces sp. C5, S. peucetius var. caesius,* and *S. bifurcus.* The basic compound of the group is daunomycin (DiMarco et al., 1964). Daunomycins may be described by the general formula I 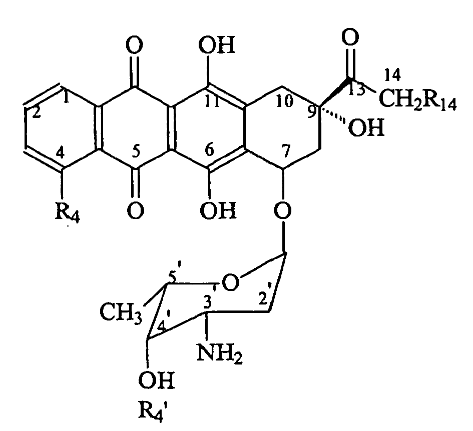 Its most important derivatives are shown in Table 1.

**Table 1**

| | | | | |
|---|---|---|---|---|
| | R₄ | R₁₄ | Others | CAS No. |
| Daunorubicin | OCH₃ | H | | 20830-81-3 |
| Epirubicin | OCH₃ | OH | 4'epi isomer | 56420-45-2 |
| Epidaunorubicin | OCH₃ | H | 4'epi isomer | 56390-08-0 |
| 13-DHED | OCH₃ | H | 4'epi isomer | |
| | | | R₁₃ = OH | |
| Idarubicin | H | H | | 58957-92-9 |

Semisynthetic derivatives of daunorubicin, epirubicin and idarubicin could be classified as unnatural anthracycline antibiotics as the natural occuring strains do not produce these anthracyclines. Epirubicin is clinically used for many types of cancer. The market is growing as it competes with doxorubicin. New formulations, conjugates and new combinations with other cancer drugs also expand the usage of epirubicin. Epirubicin is manufactured by a process which comprises producing daunorubicin by fermentation and synthetically modifying the aglycone and sugar moiety, disclosed e.g. in US Patent 5,874,550.

Idarubicin (4-demethoxy-daunorubicin) is used to treat certain types of cancer, including leukemia, lymphoma, and other diseases of the bone marrow. It is claimed to cause less side effects than doxorubicin. The global annual market for idarubicin is, however, no more than 20 kg, presumably because of its exceptionally high price, which is due to a very complicated manufacturing process. Idarubicinone is manufactured started from daunomycinone, obtained from fermentation of daunorubicin with subsequent acidic hydrolysis. Daunorubicinone is further synthetically modified to idarubicinone. A sugar residue, daunosamine, is attached by a complicated synthetic reaction series, as described in e.g. US Patent No. 4,325,946.

In fermentation production of important anthracyclines such as daunorubicin and epidaunorubicin intermediates accumulates in fermentation broth. A typical intermediate in both cases is ∈-rhodomycinone (Strohl et al., 1989). However, as epidaunorubicin may be produced by fermentation by using a genetically modified strain, 13-dihydro-epidaunorubicin (referred to as 13-DHED) is concomitantly accumulated to the fermentation broth. Typically these metabolites are considered as waste. US Patent No. 5,652,125 discloses the use of ∈-rhodomycinone to produce daunorubicin, and Dickens et al. (1997) have described the oxidization of 13-dihydro-anthracyclines to their 13-keto forms. No publications describing biotransformation in processes for un-natural anthracyclines have been located.

We have discovered a new process for obtaining higher yields of commercially important unnatural anthracyclines, epirubicin and idarubicin at lower cost by utilization of side products obtained in fermentation production of daunomycin and epidaunorubicin.

### Brief Description of the Drawings

Figure 1 is a schematic presentation of the biosynthetic pathway for daunomycin class of anthracyclines.
Figure 2 is a schematic presentation of the process for production of epirubicin and idarubicin according to this invention.
Figure 3 is a chromatogram showing the production profile of a biotransformation strain according to the present invention after feeding with 4-deoxy-∈-rhodomycinone.
   Rt = 6.0 : 13-dihydro-idarubicin, Rt = 6.9 : Idarubicin, Rt = 8.1 : 13-deoxy-Idarubicin,
   Rt = 11.0 : 4-deoxy-∈-rhodomycinone.

### Brief Description of the Invention

The present invention relates to a microbial strain of the genera Streptomyces, which converts anthracycline metabolites, such as epidaunorubicin, 13-dihydroepidauno-rubicin, 4'-epi-feudomycin and ∈-rhodomycinone, into non-natural anthracyline antibiotics, such as epirubicin and idarubicin. Preferably such strains are selected fromthe species *Streptomyces peucetius,* and most preferably from the subspecies *Streptomyces peucetius var. caesius.* In the present invention, said strain is blocked in biosynthesis of natural daunomycins and comprises a heterologous resistance gene *snor*O*,* isolated from the species *Streptomyces nogalater.*

The present invention also relates to a process for converting anthracycline metabolites such as 13-dihydroepidaunorubicin, and ∈-rhodomycinone, into non-natural anthracyline antibiotics, such as epirubicin and idarubicin using a microbial strain according to the present invention. In a preferred embodiment according to the present invention, in said process a resin, preferably selected from the group consisting of ionic and non-ionic adsorbents, more preferably from polystyrenes, and most preferably from the group consisting of XAD-7 and Diaion HP-20, is used at any time to adsorb the anthracycline metabolites or anthracycline antibiotics.

### Detailed description of the Invention

The present invention relates to improved *Streptomyces* strains, which are able to convert modified anthracycline intermediates into important antitumor anthracycline drugs, idarubicin and epirubicin. Said strains are useful in a process for converting anthracycline metabolites into the final products.

It is thus an object of the present invention to provide an improved *Streptomyces* strain, modified to be able to stably conversion of 4-deoxy-∈-rhodomycinone into idarubicin at a high rate. Such a strain, blocked to produce daunorubicin, was derived from strain G001 (DSM 12245) as described in Example 1 (Strain G001 was derived by mutagenization of wild type *S. peucetius* var. *caesius* ATCC 27952). This improved mutant strain derived from strain G001 is able to convert 4-deoxy-∈-rhodomycinone into idarubicin whereas repeatability was poor.
In order to improve conversion, resistance genes were introduced into this strain.

The gene *snor*O was isolated from S. *nogalater* (ATCC 27952) and is suggested to be responsible for the resistance to nogalamycin (Torkkell 2001). Based on sequence analysis, the gene product, SnorO is a multifunction gene product for resistance, with domains for the excision repair protein UvrA, and ABC transporter ATP-binding protein. Heterologous expression of *snor*O in *S. lividans* TK24 showed that the gene protected the strain from all tested different anthracyclines, nogalamycin, aclarubicin and daunorubicin. Introduction of the gene *snor*O into the improved strain derived from G001 provided a new biotransformation strain, which exhibits increased resistance to idarubicin. Surprisingly, *snor*O, when introduced into improved strain derived from G001 in the *E. coli* vector, improved the conversion rate and stabilized the strain to maintain the conversion within ±10%, even in six sequences of cultivations.

Said new biotransformation strain may be fed by natural anthracycline intermediates such as aklavinone and ∈-rhodomycinone and natural anthracyclines, daunomycins are formed.

Surprisingly, the strain is able to carry out conversion of unnatural metabolites and feeding with un-natural 4-deoxy-∈-rhodomycinone resulted in formation of idarubicin. Additionally, the strain is able to convert 13-DHED into epidaunorubicin. Synthetic conversion of ∈-rhodomycinone into 4-deoxy-∈-rhodomycinone is presented. In accordance with the present invention 4-deoxy-∈-rhodomycinone is subsequently biotransformed into idarubicin by the above mentioned new biotransformation strain, which does not produce daunomycin metabolites. 13-DHED was further converted into epidaunorubicin by biotransformation using the same mutant strain.

Important aspects of a biotransformation strain suitable for use in the present invention are:
i) natural and unnatural substrates are accepted for conversion;
ii) the strain has increased resistance against natural and unnatural anthracyclines;
iii) the strain accomplish essential functions needed for conversion, and
iv) the strain does not accumulate detectable quantities of natural daunomycins.

Any suitable Streptomyces strain can be used for biotransformation of the 4-deoxy-semisynthetic intermediate into idarubicin. Nevertheless, it is critical that the strain has either endogenous or transferred expressible genes for the following reactions: modifications of glucose to form daunosamine, 10-esterase activity to remove a methyl group with the connected 10-decarboxylase activity, 13-oxygenase and the suitable glycosyl transferase activity. Furthermore, the downstream process after biotransformation is cost-effective only if no or minor amounts of natural anthracycline metabolites are accumulated by the strain used as a host in biotransformation.

It is advantageous to use a *S. peucetius var. caesius* mutant blocked in the early stage of daunomycin biosynthesis; preferably a mutant blocked in minimal PKS (minimal PolyKetideSynthase catalyzes the first reactions, polyketide assembly in anthracyline and other Type II polyketide pathway).

The new biotransformation strain derived from wild type *Streptomyces peucetius var. caesius* ATCC 27952 as described above is a highly preferred strain, but any strain sharing the following characteristics is suitable for the conversion process according to the present invention.
1. Blocked in early pathway for daunomycins. Disruption of *dps*G gene of minimal PKS (data not shown) based on complementation experiment of the corresponding gene.
2. Does not produce detectable quantities of daunomycins, whereas accumulates an acidic yellow substance on solid medium. The structure of the compound is not known, but it is not a member of anthracyclines.
3. On ISP4+tsr- and ISP2+tsr-plates biotransformation strain is normally non-sporulating and forms colourless aerial hyphae.
4. Express resistance to several different anthracyclines as a consequence of *snor*O function.
5. Converts 4-deoxy-∈-rhodomycinone into two main products: idarubicin and 13-dihydroidarubicin. Some other idarubicin metabolites are found in small quantities.

The present invention further relates to a process for production of un-natural anthracycline antibiotics, epirubicin and idarubicin by exploiting the shunt products, ∈-rhodomycinone formed in the fermentation production of daunorubicin and epidaunorubicin, and 13-DHED formed in the fermentation production of epidaunorubicin.

It is thus a further object of the present invention to provide a process for preparing commercially useful anthracycline antibiotics by means of biotransformation using an improved bacterial host strain according to the present invention. Preferably, such a host strain is the new biotransformation strain described above, derived from *S. peucetius* var. *caesius.*

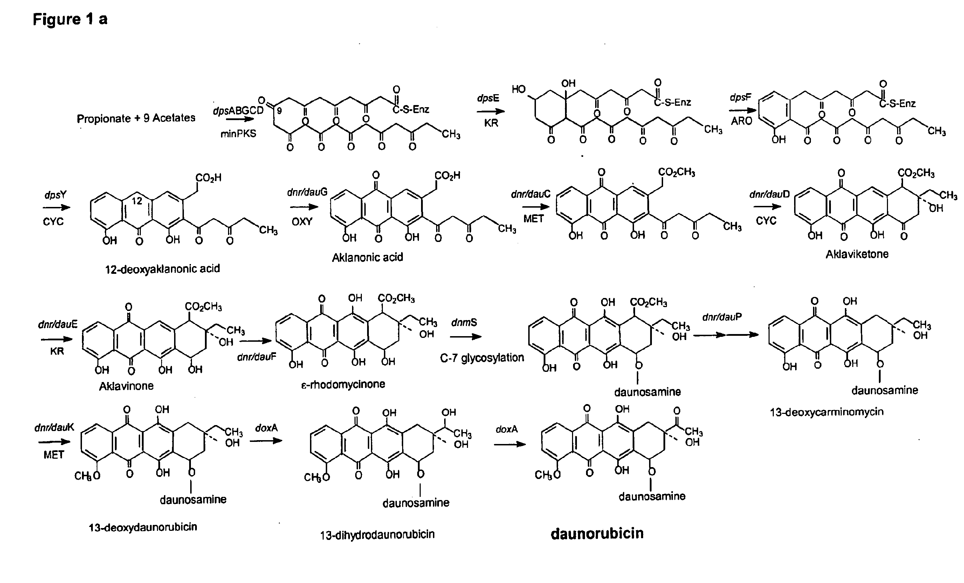

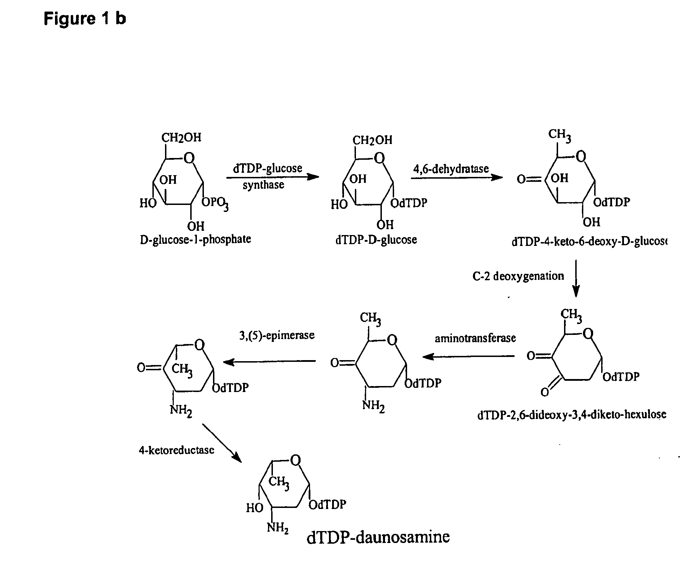

In the art, it is known that idarubicinone can be converted into idarubicin by a complicated chemical synthesis series, as described in e.g. US Patent No. 7,053,191. In the process according to the present invention an endogenous biosynthetic reaction series of a bacterial strain to convert 4-deoxy-∈-rhodomycinone into idarubicin is used. It is known that biosynthesis proceeds in the sequence shown in Fig. 1. Aklavinone, a typical precursor for several anthracyclines, is 11-hydroxylated to form ∈-rhodomycinone, which is glycosylated. The modifications in the position 10 need for a glycosylated form even though other sugar residues, such as rhodosamine, are accepted substrates. After 10-modifications, 13-oxygenation takes place and the ultimate step for daunorubicin biosynthesis is an O-methylation at C-4. Surprisingly, last 10- and 13-modifications as well as glycosylation were successful despite the 4-deoxy-form. Both 4-deoxyaklavinone and 4-deoxy-∈-rhodomycinone are converted into idarubicin using a suitable biotransformation strain. However, the gene products for these modifications need a substrate in which the substituent at C-10 is a COOCH₃-group.

∈-rhodomycinone, obtained as a shunt product by a fermentation process of daunomycin metabolites, is processed into 4-deoxy-∈-rhodomycinone by synthetic chemistry. Various synthetic paths are possible to remove a hydroxyl group from position 4 of ∈-rhodomycinone. However, we prefer to carry out the four reaction series starting by protection of C7 and C9-hydroxyl groups by ketalization. After that, trifylation of the OH-group at C4 takes place following by reduction to remove the substituent at C4. After each step the product is isolated or purified by precipitation/crystallization and the overall yield of > 20 %, preferably > 30 %, most preferably > 40 % is obtained. The purity of 4-deoxy-∈-rhodomycinone obtained in this process is > 60 %, preferably > 80 %, most preferably > 90 % being a suitable substrate for biotransformation. ∈-rhodomycinone is typically accumulated in the fermentation broth in large quantities and purification by conventional methods is successful. Synthetic conversion of ∈-rhodomycinone provides > 20 %, preferably > 30 %, most preferably > 40 % of pure 4-deoxy-∈-rhodomycinone, which is fed to the non-producing mutant strain of *S. peucetius var. caesius.* The efficiency of biotransformation of 4-deoxy-∈-rhodomycinone into idarubicin was > 20 %, preferably > 30 %, most preferably > 40 %. More than 100 mg of the semisynthetic intermediate could be fed to a litre of the culture of the biotransformation strain. The timing to feed the intermediate is not critical. Any fermentation conditions allowing growth of streptomycetes and secondary metabolism could be used whereas it is advantageous to use E1-medium and temperature range of 25-35 °C in pH between 6 to 8.

Recovery of idarubicin from the culture can be carried out with any suitable method, such as centrifuging, filtration or by a suitable ionic or non-ionic adsorbent. For insertion of idarubicin, any water-soluble organic solvent could be used, whilst acidic alcohols are preferred. Aglycones are removed with acidic extraction after which glycosides are extracted back from water phase to chloroform phase in high pH. Depending on the profile of the compounds after evaporation of the last extract, idarubicin is finally purified by crystallization or, preferably, by chromatography and crystallization. It is advantageous to use a silica column for purification.

### Conversion of 13-DHED to EPIDAUNORUBICIN

It is well known that 13-dihydro-anthracyclines accumulates to fermentation broth together with 13-keto forms. However, 13-DHED is not commercially useful and based on its cytotoxic nature, it shall be handled as a toxic waste. To our surprise, this metabolite even though not a natural one was converted to epidaunorubicin by the biotransformation strain in a useful rate, e.g. > 20 %, preferably > 30 %, most preferably > 40 %.

13-DHED, a side product in epidaunorubicin fermentation is easily separated from glycosidic fraction by chromatography followed by crystallization alongside with epidaunorubicin separation. 13-DHED could be adsorbing to a resin added to the culture broth of *Streptomyces* strain with capabilities for daunomycin synthesis and especially the late steps. Even though any strain is suitable, it is advantageous to use the strain which is blocked in early biosynthetic pathway and unable to accumulate daunomycin metabolites.

In a preferred embodiment of the present invention, the *new biotransformation strain,* derived from *S. peucetius* var. *caesius* is used for biotransformation to convert 13-DHED to epidaunorubicin.

Conversion rates in the conditions described for production of epidaunomycins according to the present invention are > 20 %, preferably > 30 %, most preferably > 40 %. Epidaunorubicin obtained in this way may be purified from other metabolites bound to the resin by any conventional methods used for anthracyclines recovery whereas chromatography and especially reverse-phase chromatography is preferably used to provide adequately purified epidaunorubicin for synthesis.

### Conversion of epidaunorubicin to epirubicin

Crude, epidaunorubicin (purity > 60 %, preferably > 80 %, most preferably > 90 %) is used as a starting material for synthetic chemistry to obtain epirubicin, which is a frequently used cancer drug. Any synthetic or biocatalytic reaction series may be used for the 14-hydroxylation.

There are several possibilities to convert epidaunorubicin into its 14-hydroxylated form, epirubicin. The endogeneous gene product alone, 14-hydroxylase, is not sufficiently active in the cultural conditions to convert all epidaunorubicin formed into epirubicin even though minor amounts of epirubicin is found in the culture broth. According to our experiments (data not shown), even high copies of the gene for 14-hydroxylase, failed to complete the process for epirubicin production. Nevertheless, two US patents, US 5,955,319 and US 6,210,930 disclose the conversion of daunorubicin into doxorubicin in a low level by the gene product of doxA. Apparently, the bioconversion is highly dependent on the conditions, and we have not succeeded in repeating the process.

There are various possibilities to add a hydroxyl group at the C-14 of the intact epidaunorubicin, analogously to synthesis of doxorubicin from daunorubicin.

Purification of epirubicin is carried out by chromatography and/or by crystallization after extraction of epirubicin from the synthesis mixture. However, to achieve the quality requested for active pharmaceutical ingredient, chromatography separation to give epirubicin in > 97 % purity is essential.

A more detailed description of the present invention is given in the examples below.

It will be obvious to a person skilled in the art that, as the technology advances, the inventive concept can be implemented in various ways. The invention and its embodiments are not limited to the examples described below but may vary within the scope of the claims.
- **Figure 1 a**: discloses a biosynthetic pathway for daunorubicin. Baumycins are formed from daunorubicin. The biosynthetic pathway is branched to (i)doxorubicin and to (ii)baumycin after daunorubicin. These steps are not shown in the figure.
- **Figure 1 b**: discloses a biosynthetic pathway for dTDP-daunosamine, which is used for the C-7-glycosylation in Fig. 1a.

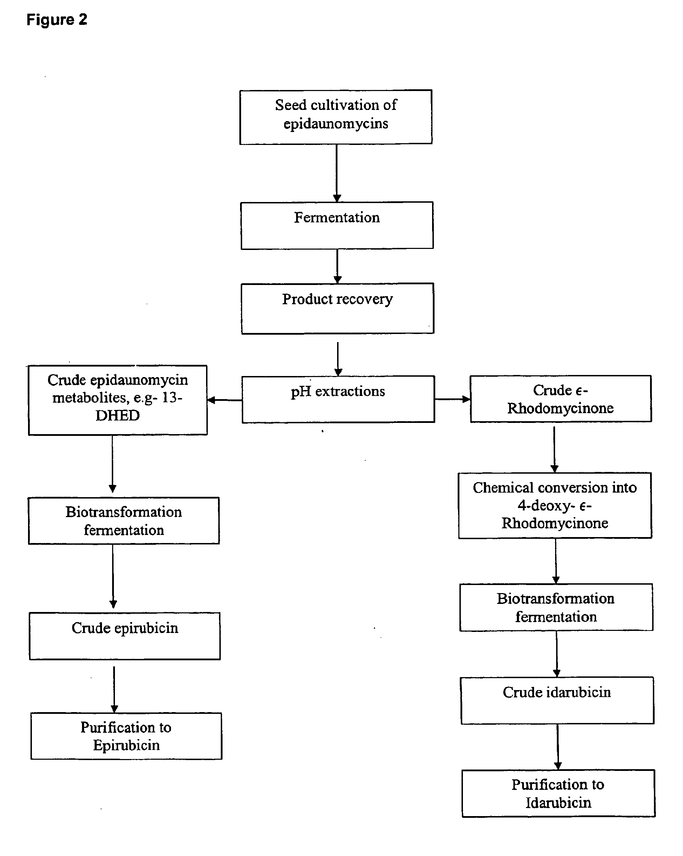

- **Figure 2**: discloses a scheme for a biotransformation process.

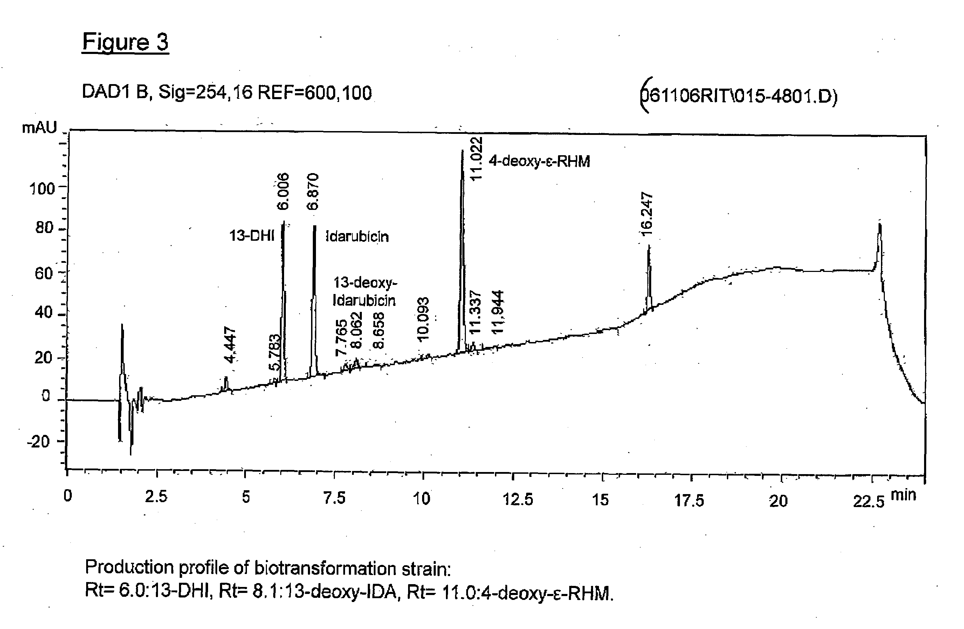

- **Figure 3**: discloses a chromatogram showing the production profile of biotransformation strain after feeding with 4-deoxy-∈-rhodomycinone.

### EXAMPLES

### Example 1 Construction of a biotransformation strain

For mutagenization strain G001, derived from wild type S. *peucetius* var. *caesius* ATCC 27952 by mutagenization was cultured in 50 ml TSB-medium in 250 ml Erlenmeyer flask. All the flasks for mutagenesis contained a string in the bottom of the flask to disperse mycelia during cultivation. Cultivation was carried out for two days at 30°C and 330 rpm in a shaker. One ml of the culture was further inoculated to the next bottle containing 50 ml TSB and cultivation was continued for one day (30°C, 330 rpm). This younger culture was adapted to alkaline pH with NaOH and NTG was added to act on the cells for 20 minutes at > 30°C. The mutagenized culture of said strain was divided into two tubes and pelleted by centrifugation (300 rpm, 10 minutes). Combined pellets were used to inoculate 50 ml of TSB medium. After one day (30°C, 330 rpm), the titre of the cell suspension was determined by plating suitable dilutions, said 1:10 - 1: 100000 on ISP4-plates. Colonies of mutagenized cultures were compared to the wild type and those exhibiting distinct features from the wild type were selected for further characterization.

The mutants were selected based on pale colour from the dark red-pigmented wild type on the ISP4 -agar plate.

The gene *snor*O (Torkkell, 2001) was introduced into the mutants with *E.coli* vector, pCNB3033. Integration was suggested to occur by *att*P-site. The obtained improved biotransformation strain gave repeated conversion rates for > 20 %, preferably > 30 %, most preferably > 40 % of fed 4-deoxy-∈-rhodomycinone into idarubicin metabolites as is detailed described in Example 5.

### Example 2 Cultivation of biotransformation strains for production of anthracyline metabolites and for biotransformation

Suitable mutants selected as described in example 1 were cultivated in 50 ml of E1-medium supplemented with adsorbent resin (15 g/l). (E1: Per litre of tap water: glucose 20 g; soluble starch 20 g; Peptide 5 g; Yeast extract 2.5 g; K₂HPO₄·3H₂O 1.3 g; MgSO₄·7H₂O 1 g; NaCl 3 g; CaCO₃ 3 g; pH 7-7.5).

To determine the anthracycline metabolites the compounds were extracted at the tenth day of incubation. For analysis, the adsorbent resin was decanted with water from one cultivation flask and washed resin was extracted with 40 ml of acidic alcohol shaking for at least 30 min. The HPLC was used for analysing the samples. The use of adsorbent resin in E1-medium was known to increase production of anthracycline metabolites in said conditions.
The changes in colony morphology were not found at the end of cultivation in the presence of the adsorbent.

Production of anthracycline metabolites were not detected in culture broth of the mutant strains.

However, mutant strains have the functional biosynthetic genes for the glycosylation and modification of aglycones according to biosynthesis pathway of Figure 1 and as was demonstrated by feeding experiments. The natural aglycones, ∈-rhodomycinone and aklavinone were fed to the mutant strains (at least 100 mg of the aglycone per 1 litre of the culture broth) and the fed aglycone was converted into daunomycin metabolites in two days. However, feeding the mutant strains with 4-deoxy-∈-rhodomycinone failed to give repeated conversion to idarubicin metabolites. The reason for the failure was suggested to be caused by a poor resistance against fed or formed product.

The improved biotransformation strain was able to convert natural aglycones, ∈-rhodomycinone and aklavinone as was expected. It also converted un-natural analogous biosynthetic intermediate, 4-deoxy-∈-rhodomycinone and 13-DHED to idarubicin and to epidaunorubicin, as is described in the example 4 below.

### Example 3 Synthetic conversion of ∈-rhodomycinone into 4-deoxy-∈-rhodomycinone

Purified ∈-rhodomycinone of > 60 %, preferably > 80 %, most preferably > 90 % chromatographic purity was in use for synthesis of 4-deoxy-∈-rhodomycinone.
The synthesis of 4-deoxy-∈-rhodomycinone consists of four steps:
A) protection, B) trifylation, C) reduction and D) deprotection.

After each step the product was isolated by precipitation/crystallization from chloroform-methanol mixtures. Synthesis reactions were monitored with TLC.

### A) Protection

∈-rhodomycinone was dissolved in chloroform at room temperature. 2 eqv. of 2-methoxypropene was added to a mixture followed by 1 eqv. of TMSCI. Reaction was followed by TLC. When the reaction was finished as was detected on TLC plate, the mixture was allowed to cool down and the precipitate was collected and dried for the next step.

### B) Trifylation

A starting material from protection step was dissolved in chloroform. NMP (N-methyl pyrrolidone) and di-isopropylethylamine (DIPEA) were added and finally PhNTf2. Reaction was monitored with TLC. The compound was precipitated by adding water and citric acid to mixture. Precipitate was filtered and washed with KHSO₄. Crystals were filtered and dried under vacuum.

### C) Reduction

Material from trifylation was suspended to ACN under argon. (Ph3P)4Ph was added followed by addition of (Ethyl)₃N and HCOOH. Reaction mixture was heated up and reaction was followed by TLC. After 1 h reaction was completed. Reaction mixture was allowed to cool down and the product was filtered off.

### D) Deprotection

The product from reduction was dissolved in chloroform and 1 eqv. of TsOH x H₂O was added at RT. Reaction proceeds within 0.5 h. Solution was washed with 1 M NaHCO₃, dried with MgSO₄ and evaporated to dryness.

### 4-deoxy-∈-rhodomycinone purification

A deprotection reaction mixture (CHCl₃ / TsOH x H₂O) was diluted with chloroform, and washed with 1 M NaHCO₃. The chloroform fraction was dried with anhydrous MgSO4, filtered and evaporated to dryness. Crystallization was done from chloroform-methanol. Crystals were filtered and dried under vacuum.

### Example 4 Recovery of 13-DHED from culture broth of a epidaunorubicin-producing mutant strain derived from Streptomyces peucetius var. caesius

Fermentation broth for production of epidaunorbicin contains three major metabolites in this order: Epidaunorubicin, 13-DHED and epi-feudomycin (Ref. epi-patent application). The substituents adsorbed to a resin were decanted from the 20 litre culture broth obtained from fermentation. The resin was washed to remove cell debris by water. Pellet was extracted with alcohol for one to five times. The aglycones were extracted with chloroform by adding chloroform to the combined alcohol extracts. Solvent and water layers were separated. Glycosides in water phase were extracted to chloroform at lightly alkaline pH and pH was stabilized with saturated NaHCO₃. Salts were removed by washing with water. Finally the chloroform-phase was filtrated through a cartridge filter.

Silica gel chromatography was carried out to separate the metabolites. The filtrated chloroform was pumped into a silica column and purified by chromatography using chloroform-methanol-solution as a mobile phase. Pure fractions of each three metabolites were collected, and fractions of each product were pooled for crystallization. The fractions of 13-DHED were crystallized by ethanol-water solutions. The crystals were filtrated and adsorbed to adsorbent resin.

### Example 5 Biosynthetic conversion of 4-deoxy-∈-rhodomycinone and related metabolites into idarubicin

Seed culture was made by cultivating a biotransformation strain in two flasks of 400 ml of the E1 medium for three days. The cultures were combined and the 800 ml of the culture broth were used to inoculate a 20 litre E1-medium supplemented with XAD-7. Fermentation was carried out in 20 litres volume for 8 days at the temperature of 30 °C, 350 rpm with the aeration of 10 l/min. pH has to be kept slightly acid, which ensures more stable conversion of fed 4-deoxy-∈-rhodomycinone into idarubicin. 4-deoxy-∈-rhodomycinone is feeded continously 4 days started 24 hours after inoculation with at least 5 mg/ml 4-deoxy-∈-rhodomycinone in EtOH. Feeded 4-deoxy-∈-rhodomycinone amount is at least 100 mg/l.

During biotransformation with mutant strain two main secondary metabolites were produced from the fed 4-deoxy-∈-rhodomycinone; namely idarubicin and 13-dihydro-idarubicin. Other minor metabolites were idarubicinone, 13-dihydroidarubicinone, baumycins and 13-deoxy-Idarubicin and its aglycone. Stable titre of Idarubicin is > 20 mg/L, in addition > 20 mg/L of previous intermediate 13-DHI is produced; thus altogether the amount is > 40 mg/L. Baumycins can be hydrolysed to Idarubicin by heating in acidic conditions (+45 °C, 1 hour).
The chromatograph of the products obtained from biotransformation strain is shown in Figure 3.

### Example 6 Biosynthetic conversion of 13-DHED into epidaunorubicin

Pre-cultivation of biotransformation strain was done as detailed described in Example 5.
13-DHED adsorbed to resin corresponding to at least 50 mg/ l of the culture broth was added to the cultivation after one day and cultivation was continued for four days. Fermentations were carried out in flasks containing 50 ml E1-medium, at 34 °C, 300 rpm.

According to the chromatographic analysis, 50 % of 13-DHED was converted to epidaunorubicin. Minor amounts of epirubicin, in the range of < 10 %, were detected.

### Example 7 Analytical measurement of aglycones and anthracyclines

### TLC

1. 0.5 MQ-water
2. 0.1 HCOOH
3. 25 MeOH
4. 75 CHCl₃, mix with solutions 1, 2 and 3 carefully

### HPLC

Equipment: Hewlett-Packard chromatography equipment belonging to series 1100 with diode array detector.
Column: Zorbax, SB-C8, Agilent, 4,6 x 150 mm 3,5-Micron
Solvents used: 0.05 % TFA, and 1:1 MeCN - tetrahydrofuran
Temperature of the column: 30°C
Stream velocity: 1 ml/min
Detection: 254±8 nm, reference wavelength 600 nm±50 nm
Injection volume: 5 µl
Pressure: min 20 bar, max 300 bar

### Parameters used:

| **time (min)** | **%-amount of 0,05 % TFA** | **%-amount of MeCN-THF** |
|---|---|---|
| 0 | 76,0 | 24,0 |
| 0,1 | 76,0 | 24,0 |
| 13,0 | 32,0 | 68,0 |
| 16,0 | 1,0 | 99,0 |
| 19,0 | 1,0 | 99,0 |
| 20,0 | 76,0 | 24,0 |
| 24,0 | 76,0 | 24,0 |

### List of References

Dickens M, Priestley N and Strohl W: In vivo and in vitro bioconversion of ∈-rhodomycinone glycoside to doxorubicin: Function of DauP, DauK, and DoxA. (1997) J. Bacteriol. 179: 2641-2650.
Di Marco A, Silvestrini R, Gaetani M, Soldati M, Orezzi P, Dasdia T, Scarpinato BM, Valentini L: 'daunomycin', a new antibiotic of the rhodomycin group. (1964) Nature 15: 706-707.
Hopwood DA, Bibb MJ, Chater KF, Kieser T, Bruton CJ, Kieser HM, Lydiate DJ, Smith CP, Ward JM and Schrempf H: Genetic Manipulation of Streptomyces: a Laboratory Manual. (1985) John Innes Foundation, Norwich.
Sambrook J, Fritsch EF and Maniatis T: Molecular Cloning: a Laboratory Manual. (1989) Cold Spring Harbor Laboratory, Cold Spring Harbor, NY.
Strohl W.R, Bartel P, Connors NC, Zhu C, Dosch D, Beale JM, Floss HG, Stuzman-Engwall K, Otten SL and Hutchinson CR: Biosynthesis of natural and hybrid polyketides by anthracycline-producing streptomycetes. (1989) p. 68-84, C.L. Hershberger, SW Queener and G Hegeman (ed.) Genetics and molecular biology of industrial micro-organisms. American Society for Microbiology, Washington, D.C.
Torkkell S, Kunnari T, Palmu K, Mäntsälä P, Hakala J and Ylihonko K: The entire nogalamycin biosynthetic gene cluster of Streptomyces nogalater: characterization of a 20-kb DNA region and generation of hybrid structures. (2001) Molecular Genetics and Genomics 266:276-288.
Torkkell S: Anthracycline antibiotics: Biosynthetic pathway and molecular genetics of nogalamycin, a product of Streptomyces nogalater. (2001) Publications in Annales Universitatis Turkuensis-series nr: 275.

## Claims

1. A microbial strain selected from the genera *Streptomyces* which converts anthracycline metabolites into non-natural anthracyline antibiotics, **characterized in that** the strain is blocked in biosynthesis of natural daunomycins and comprises heterologous resistance gene snorO.

2. The strain according to claim 1, wherein said anthracycline metabolites are selected from the group consisting of epidaunorubicin, 13-dihydroepidaunorubicin, 4'-epi-feudomcyin and ε-rhodomycinone.

3. The strain according to any one of claims 1 or 2, wherein said non-natural anthracycline antibiotics are selected from the group consisting of epirubicin and idarubicin.

4. The strain according to claim 1, wherein said strain is selected from the species *Streptomyces peucetius.*

5. The strain according to claim 4, wherein said strain is selected from the subspecies *Streptomyces peucetius* var *caesius.*

6. The strain according to claim 1, wherein said gene is isolated from genus *Streptomyces.*

7. The strain according to claim 6, wherein said gene is isolated from the species *Streptomyces nogalater.*

8. The strain according to any one of claims 1 to 7, wherein said strain provides a conversion rate of the anthracycline metabolites into the non-natural anthracycline antibiotics of > 20 %.

9. The strain according to claim 8, wherein said strain provides a conversion rate of the anthracycline metabolites into the non-natural anthracycline antibiotics of > 30 %.

10. The strain according to claim 9, wherein said strain provides a conversion rate of the anthracycline metabolites into the non-natural anthracycline antibiotics of > 40 %.

11. A process for converting anthracycline metabolites into anthracycline antibiotics using a microbial strain according to any one of claims 1 to 10.

12. The process according to claim 11, wherein said anthracycline metabolites are selected from the group consisting of epidaunorubicin, 13-dihydroepidaunorubicin, 4'-epi-feudomcyin and ε-rhodomycinone.

13. The process according to claim 11 or 12, wherein said anthracycline antibiotics are selected from the group consisting of epirubicin and idarubicin.

14. The process according to any one of claims 11 to 13, wherein said strain provides a conversion rate of the anthracycline metabolites into the non-natural anthracycline antibiotics of > 20%.

15. The process according to claim 14, wherein said strain provides a conversion rate of the anthracycline metabolites into the non-natural anthracycline antibiotics of > 30 %.

16. The process according to claim 15, wherein said strain provides a conversion rate of the anthracycline metabolites into the non-natural anthracycline antibiotics of > 40 %.

17. The process according of any one of claims 11 to 16, wherein purity of crude anthracycline metabolites used for biotransformation is > 60 %.

18. The process according to claim 17, wherein purity of crude anthracycline metabolites used for biotransformation is > 80 %.

19. The process according to claim 18, wherein purity of crude anthracycline metabolites used for biotransformation is > 90 %.

20. The process according to any one of claims 11 to 19, wherein a resin is used at any time to adsorb the anthracycline metabolites or anthracycline antibiotics.

21. The process according to claim 20, wherein said resin is selected from the group consisting of ionic and non-ionic adsorbents.

22. The process according to claim 21, wherein said resin is selected from the group of polystyrenes.

23. The process according to claim 21, wherein said resin is selected from the group consisting of XAD-7 and Diaion HP-20.

24. The process according to any one of claims 21 to 23, wherein said resin is added in an amount of 1 - 100 g/l.

25. The process according to claim 24, wherein said resin is added in an amount of 10 - 50 g/l.

26. The process according to claim 25, wherein said resin is added in an amount of 15 - 30 g/l.

## Patentansprüche

1. Mikrobenstamm, ausgewählt aus der Gattung *Streptomyces,* der Anthracyclinmetaboliten in nicht-natürliche Anthracyclinantibiotika umwandelt, **dadurch gekennzeichnet, dass** die Biosynthese von natürlichen Daunomycinen bei dem Stamm blockiert ist und der Stamm heterologes Resistenzgen snorO umfasst.

2. Stamm nach Anspruch 1, wobei die Anthracyclinmetaboliten aus der aus Epidaunorubicin, 13-Dihydroepidaunorubicin, 4'-Epi-Feudomycin und ε-Rhodomycinon bestehenden Gruppe ausgewählt sind.

3. Stamm nach einem der Ansprüche 1 oder 2, wobei die nicht-natürlichen Anthracyclinantibiotika aus der aus Epirubicin und Idarubicin bestehenden Gruppe ausgewählt sind.

4. Stamm nach Anspruch 1, wobei der Stamm aus der Art *Streptomyces peucetius* ausgewählt ist.

5. Stamm nach Anspruch 4, wobei der Stamm aus der Unterart *Streptomyces peucetius* var. *caesius* ausgewählt ist.

6. Stamm nach Anspruch 1, wobei das Gen aus der Gattung *Streptomyces* isoliert ist.

7. Stamm nach Anspruch 6, wobei das Gen aus der Art *Streptomyces nogalater* isoliert ist.

8. Stamm nach einem der Ansprüche 1 bis 7, wobei der Stamm eine Umwandlungsrate für die Anthracyclinmetaboliten in die nicht-natürlichen Anthracyclinantibiotika von > 20% bereitstellt.

9. Stamm nach Anspruch 8, wobei der Stamm eine Umwandlungsrate für die Anthracyclinmetaboliten in die nicht-natürlichen Anthracyclinantibiotika von > 30% bereitstellt.

10. Stamm nach Anspruch 9, wobei der Stamm eine Umwandlungsrate für die Anthracyclinmetaboliten in die nicht-natürlichen Anthracyclinantibiotika von > 40% bereitstellt.

11. Prozess zur Umwandlung von Anthracyclinmetaboliten in Anthracyclinantibiotika unter Verwendung eines Mikrobenstamms nach einem der Ansprüche 1 bis 10.

12. Prozess nach Anspruch 11, wobei die Anthracyclinmetaboliten aus der aus Epidaunorubicin, 13-Dihydroepidaunorubicin, 4'-Epi-Feudomycin und ε-Rhodomycinon bestehenden Gruppe ausgewählt sind.

13. Prozess nach Anspruch 11 oder 12, wobei die Anthracyclinantibiotika aus der aus Epirubicin und Idarubicin bestehenden Gruppe ausgewählt sind.

14. Prozess nach einem der Ansprüche 11 bis 13, wobei der Stamm eine Umwandlungsrate für die Anthracyclinmetaboliten in die nicht-natürlichen Anthracyclinantibiotika von > 20% bereitstellt.

15. Prozess nach Anspruch 14, wobei der Stamm eine Umwandlungsrate für die Anthracyclinmetaboliten in die nicht-natürlichen Anthracyclinantibiotika von > 30% bereitstellt.

16. Prozess nach Anspruch 15, wobei der Stamm eine Umwandlungsrate für die Anthracyclinmetaboliten in die nicht-natürlichen Anthracyclinantibiotika von > 40% bereitstellt.

17. Prozess nach einem der Ansprüche 11 bis 16, wobei die Reinheit von zur Biotransformation verwendeten Roh-Anthracyclinmetaboliten > 60 % beträgt.

18. Prozess nach Anspruch 17, wobei die Reinheit von zur Biotransformation verwendeten Roh-Anthracyclinmetaboliten > 80 % beträgt.

19. Prozess nach Anspruch 18, wobei die Reinheit von zur Biotransformation verwendeten Roh-Anthracyclinmetaboliten > 90 % beträgt.

20. Prozess nach einem der Ansprüche 11 bis 19, wobei ein Harz zu einem beliebigen Zeitpunkt zur Adsorption der Anthracyclinmetaboliten oder Anthracyclinantibiotika verwendet wird.

21. Prozess nach Anspruch 20, wobei das Harz aus der aus ionischen und nichtionischen Adsorptionsmitteln bestehenden Gruppe ausgewählt wird.

22. Prozess nach Anspruch 21, wobei das Harz aus der Gruppe der Polystyrole ausgewählt wird.

23. Prozess nach Anspruch 21, wobei das Harz aus der aus XAD-7 und Diaion HP-20 bestehenden Gruppe ausgewählt wird.

24. Prozess nach einem der Ansprüche 21 bis 23, wobei das Harz in einer Menge von 1 - 100 g/l zugesetzt wird.

25. Prozess nach Anspruch 24, wobei das Harz in einer Menge von 10 - 50 g/l zugesetzt wird.

26. Prozess nach Anspruch 25, wobei das Harz in einer Menge von 15 - 30 g/l zugesetzt wird.

## Revendications

1. Souche microbienne sélectionnée parmi le genre *Streptomyces* qui convertit les métabolites des anthracyclines en antibiotiques anthracyclines non naturelles, **caractérisée en ce que** la souche est bloquée dans la biosynthèse des daunomycines naturelles et comprend le gène de résistance hétérologue snorO.

2. Souche selon la revendication 1, où lesdits métabolites des anthracyclines sont sélectionnés dans le groupe constitué de l'épidaunorubicine, la 13-dihydroépidaunorubicine, la 4'-épi-feudomycine et la ε-rhodomycinone.

3. Souche selon l'une quelconque des revendications 1 ou 2, où lesdits antibiotiques anthracyclines non naturelles sont sélectionnés dans le groupe constitué de l'épirubicine et de l'idarubicine.

4. Souche selon la revendication 1, où ladite souche est sélectionnée parmi l'espèce *Streptomyces peucetius.*

5. Souche selon la revendication 4, où ladite souche est sélectionnée parmi la sous-espèce *Streptomyces peucetius* var. *caesisus.*

6. Souche selon la revendication 1, où ledit gène est isolé du genre *Streptomyces.*

7. Souche selon la revendication 6, où ledit gène est isolé de l'espèce *Streptomyces nogalater.*

8. Souche selon l'une quelconque des revendications 1 à 7, où ladite souche assure un taux de conversion des métabolites des anthracyclines en les antibiotiques anthracyclines non naturelles > 20 %.

9. Souche selon la revendication 6, où ladite souche assure un taux de conversion des métabolites des anthracyclines en les antibiotiques anthracyclines non naturelles > 30 %.

10. Souche selon la revendication 9, où ladite souche assure un taux de conversion des métabolites des anthracyclines en les antibiotiques anthracyclines non naturelles > 40 %.

11. Procédé de conversion des métabolites des anthracyclines en antibiotiques anthracyclines au moyen d'une souche microbienne selon l'une quelconque des revendications 1 à 10.

12. Procédé selon la revendication 11, où lesdits métabolites des anthracyclines sont sélectionnés dans le groupe constitué de l'épidaunorubicine, de la 13-dihydroépidaunorubicine, de la 4'-épi-feudomycine et de la ε-rhodomycinone.

13. Procédé selon la revendication 11 ou 12, où lesdits antibiotiques anthracyclines sont sélectionnés dans le groupe constitué de l'épirubicine et de l'idarubicine.

14. Procédé selon l'une quelconque des revendications 11 à 13, où ladite souche assure un taux de conversion des métabolites des anthracyclines en les antibiotiques anthracyclines non naturelles > 20 %.

15. Procédé selon la revendication 14, où ladite souche assure un taux de conversion des métabolites des anthracyclines en les antibiotiques anthracyclines non naturelles > 30 %.

16. Procédé selon la revendication 15, où ladite souche assure un taux de conversion des métabolites des anthracyclines en les antibiotiques anthracyclines non naturelles > 40 %.

17. Procédé selon l'une quelconque des revendications 11 à 16, où la pureté des métabolites bruts des anthracyclines utilisés pour la biotransformation est > 60 %.

18. Procédé selon la revendication 17, où la pureté des métabolites bruts des anthracyclines utilisés pour la biotransformation est > 80 %.

19. Procédé selon la revendication 18, où la pureté des métabolites bruts des anthracyclines utilisés pour la biotransformation est > 90 %.

20. Procédé selon l'une quelconque des revendications 11 à 19, où une résine est utilisée à un moment quelconque pour adsorber les métabolites des anthracyclines ou les antibiotiques anthracyclines.

21. Procédé selon la revendication 20, où ladite résine est sélectionnée dans le groupe constitué des absorbants non ioniques et ioniques.

22. Procédé selon la revendication 21, où ladite résine est sélectionnée dans le groupe des polystyrènes.

23. Procédé selon la revendication 21, où ladite résine est sélectionnée dans le groupe constitué de XAD-7 et Diaion HP-20.

24. Procédé selon l'une quelconque des revendications 21 à 23, où ladite résine est ajoutée en une quantité de 1 à 100 g/l.

25. Procédé selon la revendication 24, où ladite résine est ajoutée en une quantité de 10 à 50 g/l.

26. Procédé selon la revendication 25, où ladite résine est ajoutée en une quantité de 15 à 30 g/l.
